# FASCICULE DE BREVET EUROPEEN

(11) **EP 2 391 707 B1**
(45) Date de publication et mention de la délivrance du brevet: **23.07.2014**
(21) Numéro de dépôt: 10704997.5
(22) Date de dépôt: 26.01.2010
(51) Int. Cl.: C12N 1/18, A01N 63/00, C12R 1/865

(54) **SOUCHES DE SACCHAROMYCES CEREVISIAE A APTITUDES PHYTOSANITAIRES**
SACCHAROMYCES CEREVISIAE-STÄMME MIT PFLANZENSCHUTZFÄHIGKEITEN
SACCHAROMYCES CEREVISIAE STRAINS WITH PHYTOSANITARY CAPABILITIES

(30) Priorité: 27.01.2009 FR 0900339
(43) Date de publication de la demande: 07.12.2011
(73) Titulaire: Lesaffre et Compagnie, 75001 Paris (FR)
(72) Inventeur: PUJOS, Philippe, 59000 Lille (FR); COLAVIZZA, Didier, 59100 Roubaix (FR); VANDEKERCKOVE, Pascal, 59650 Villeneuve d'Ascq (FR)
(74) Mandataire: Cabinet Plasseraud
(86) Numéro de dépôt international: PCT/IB2010/050336
(87) Numéro de publication internationale: WO 2010/086790

(56) Documents cités:
- WO-A1-2006/032530
- FR-A1- 2 894 771

## Description

### DOMAINE DE L'INVENTION

La présente invention concerne des levures *Saccharomyces cerevisiae* utiles en tant que microorganismes antagonistes pour protéger les plantes contre divers agents pathogènes, notamment des champignons. L'invention est adaptée au traitement de multiples variétés végétales.

### ARRIERE-PLAN TECHNIQUE

Parmi les agents pathogènes des végétaux, les champignons, responsables des maladies fongiques ou cryptogamiques, sont ceux qui ont le plus grand impact économique. Chaque espèce végétale est sensible à une ou plusieurs maladies principales, susceptibles de réduire fortement sa vigueur, sa croissance, et finalement la quantité ou/et la qualité de la récolte.

Divers paramètres influencent le développement des maladies comme les conditions de sol et la fertilisation, la sensibilité variétale, le mode de conduite (rotation, travail du sol, nombre de plantes ou plants par hectare, système de taille...), ou surtout les conditions climatiques. Mais agir sur certains de ces paramètres ne suffit généralement pas à limiter suffisamment les dégâts occasionnés par les maladies. Aussi, pour se prémunir, le praticien souhaitant optimiser et sécuriser son rendement traitera sa culture au moment opportun avec un produit phytosanitaire, souvent préventif. Le plus souvent les produits utilisés sont des produits chimiques, pour la plupart très efficaces, mais pouvant présenter des risques sanitaires pour les personnels qui les manipulent, et générant des résidus sur les productions traitées, dans les sols et les eaux. De plus, l'utilisation répétée de certaines matières actives fongicides agissant sur le même site métabolique sélectionne des souches résistantes à ces fongicides.

Pour tenter d'y remédier, il est nécessaire de limiter le nombre d'utilisations annuelles de produits chimiques de la même famille, d'alterner les familles chimiques à modes d'action différents, et d'utiliser tous autres moyens défavorables à l'agent pathogène. Dans ce contexte, il existe donc un besoin réel et important de solutions alternatives contre les maladies des plantes.

Idéalement, ces solutions doivent agir de manière différente de celle des fongicides chimiques existants, ne pas générer de résidus chimiques dans les récoltes et dans l'environnement, et être plus sûres et saines pour les opérateurs. De tels traitements doivent être utilisés, seuls ou en alternance et / ou combinaison avec les traitements chimiques actuels ou tout autre traitement, pour empêcher l'apparition ou limiter le développement de ces pathogènes, et de leurs souches résistantes, sur les végétaux, et limiter les risques pour l'homme et l'environnement.

Dans ce contexte, il est connu d'administrer aux végétaux des microorganismes antagonistes vis-à-vis des agents pathogènes. Par exemple, des microorganismes proposés jusqu'ici en tant que microorganismes antagonistes comprennent la bactérie *Bacillus subtilis,* les champignons *Trichoderma harzanium, Trichoderma viride, Coniothyrium minitans, Streptomyces griseoviridis,* les levures *Aureobasidium pullulans, Metschnikowia fructicola, Candida oleophila...* L'article *Biological control of postharvest diseases of fruits and vegetable* par El Ghaouth et al. (Applied Mycology and Biotechnology, vol.2, Agriculture and food production, Elsevier Science B.V., p.219-238) propose une revue des connaissances en matière de contrôle biologique des agents pathogènes sur les fruits, et propose plusieurs explications possibles pour le mode d'action des microorganismes antagonistes.

Il a été également envisagé d'utiliser des levures *Saccharomyces cerevisiae* en tant que microorganismes antagonistes (WO 2006/032530). Cependant, en général l'efficacité de ces levures a été jugée comme étant inférieure à celle d'autres microorganismes, voire dans certains cas nulle. A titre d'exemple, on pourra se reporter à l'article *Role of competition for sugars in yeasts in the biocontrol if gray mold in apples,* par A.B. Filonow (Biocontrol Science and Technology, 8:243-256, 1998), qui enseigne que *Saccharomyces cerevisiae* n'est pas efficace pour réduire l'infection des pommes par la pourriture grise, à la différence de *Cryptococcus laurentii* et de *Sporobolomyces roseus.*

Or, les microorganismes antagonistes habituellement considérés comme les plus efficaces sont souvent isolés du milieu naturel, sont peu connus, et peuvent de ce fait exprimer des caractéristiques non souhaitées lors de leur développement ou lors de leur utilisation. On se heurte alors à des difficultés d'industrialisation, voire à des inconvénients vis-à-vis des humains ou de l'environnement.

Il existe donc un besoin de mettre au point de nouvelles souches de microorganismes antagonistes, plus fiables d'un point de vue industriel, plus sûres d'un point de vue sanitaire et environnemental, et efficaces pour lutter contre les maladies provoquées par les agents pathogènes chez les végétaux.

### RESUME DE L'INVENTION

L'invention concerne en premier lieu une souche de *Saccharomyces cerevisiae,* caractérisée en ce qu'elle est choisie parmi la souche n°I-3936 déposée le 4 mars 2008 auprès de la CNCM (Collection nationale de cultures de microorganismes, Institut Pasteur, 25 rue du Docteur Roux, 75724 Paris Cedex 15, France), la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM.

L'invention concerne également une composition phytosanitaire, comprenant des levures *Saccharomyces cerevisiae* choisies parmi la souche n°I-3936 déposée le 4 mars 2008 auprès de la CNCM, la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM et les mélanges de celles-ci.

Selon un mode de réalisation, la composition phytosanitaire comprend également un ou plusieurs agents de formulation et / ou au moins un adjuvant actif supplémentaire.

Selon un mode de réalisation, la composition phytosanitaire est une composition phytosanitaire concentrée, à formulation sèche solide ou liquide.

Selon un mode de réalisation, la composition phytosanitaire est une composition phytosanitaire prête à l'emploi à formulation sèche ou liquide, de préférence liquide.

Selon un mode de réalisation, la composition phytosanitaire comprend une quantité de levures *Saccharomyces cerevisiae* comprise entre 10⁴ et 10¹¹ ufc/mL, de préférence entre 10⁵ et 10¹⁰ ufc/mL, de manière plus particulièrement préférée entre 5×10⁵ et 5×10⁹ ufc/mL.

L'invention concerne également un procédé de traitement ou de protection de végétaux contre des maladies provoquées par des agents pathogènes, comprenant la mise en contact des végétaux avec des levures *Saccharomyces cerevisiae* choisies parmi la souche n°I-3936 déposée le 4 mars 2008 auprès de la CNCM, la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM et les mélanges de celles-ci.

Selon un mode de réalisation, les agents pathogènes sont choisis parmi les champignons, virus, bactéries, mycoplasmes, spiroplasmes, viroïdes et combinaisons de ceux-ci, de préférence parmi les champignons.

Selon un mode de réalisation, les agents pathogènes sont choisis parmi les microorganismes des genres *Alternaria* spp., notamment *Alternaria solani et Alternaria alternata, Ascochyta spp., notamment Ascochyta fabae ou Ascochyta pinodella, Aspergillus spp., notamment Aspergillus niger et Aspergillus fumigatus, Botrytis spp., notamment Botrytis cinerea, Bremia spp., notamment Bremia* lactucae, *Cercospora* spp., notamment *Cercospora beticola, Cladosporium* spp., notamment *Cladosporium allii-cepae, Colletotrichum spp.,* notamment *Colletotrichum graminicola, Cryptosporiopsis spp notamment Cryptosporiopsis malicorticis, Erysiphe* spp., notamment *Erysiphe graminis ou necator, Fusarium* spp., notamment *Fusarium oxysporum* et *Fusarium roseum, Gloeosporium* spp., notamment *Gloeosporium fructigenum, Guignardia* spp., notamment *Guignardia bidwellii, Helminthosporium* spp., notamment *Helminthosporium tritici-repentis, Marssonina* spp., notamment *Marssonina rosae, Monilia* spp., notamment *Monilia fructigena, Mycosphaerella* spp., notamment *Mycosphaerella brassicicola, Penicillium* spp., notamment *Penicillium expansum* ou *Penicillium digitatum, Peronospora* spp., notamment *Peronospora parasitica, Pezicula spp. notamment Pezicula malicorticis, Phragmidium* spp., notamment *Phragmidium rubi-idaei, Phytophtora* spp., notamment *Phytophtora infestans, Plasmopara spp.,* notamment *Plasmopara viticola, Podosphaera* spp., notamment *Podosphaera leucotricha, Pseudocercosporella* spp., dont *Pseudocercosporella brassicae, Pseudoperonospora* spp., notamment *Pseudoperonospora cubensis, Pseudopeziza* spp., notamment *Pseudopeziza medicaginis, Puccinia* spp., notamment *Puccinia graminis, Pythium* spp., *Ramularia spp.,* notamment *Ramularia betae, Rhizoctonia* spp., notamment *Rhizoctonia solani, Rhizopus spp.,* notamment *Rhizopus nigricans et Rhizopus stolonifer, Rynchosporium spp.,* notamment *Rynchosporium secalis, Sclerotinia* spp., notamment *Sclerotinia sclerotiorum, Septoria* spp., notamment *Septoria nodorum* ou *Septoria tritici, Sphaerotheca spp.,* notamment *Sphaerotheca macularis, Spilocaea spp notamment Spilocaea pomi, Taphrina* spp., notamment *Taphrina pruni, Trichothecium spp. notamment roseum, Uncinula* spp., notamment *Uncinula necator, Ustilago* spp., notamment *Ustilago tritici, Venturia* spp., notamment *Venturia inaequalis,* et les combinaisons de ceux-ci, lesdits agents pathogènes étant de préférence choisis parmi *Penicillium digitatum, Penicillium expansum, Botrytis cinerea* et les combinaisons de ceux-ci.

Selon un mode de réalisation, les végétaux sont choisis parmi les graminées, les dicotylédones, les plantes annuelles, bisannuelles et pérennes, les plants de légumes ou les légumes récoltés, les plantes ou arbres à fruits ou les fruits récoltés, les plantes ou arbres à fleurs ou les fleurs récoltées, les céréales, les oléagineux, les protéagineux, les plantes ligneuses, les végétaux d'ornement, et sont notamment choisis parmi les plants ou produits issus de pomme de terre, betterave, canne à sucre, tabac, vigne, blé, colza, orge, riz, maïs, sorgho, millet, soja, haricot, tomate, concombre, laitue, fraisier, pommier, poirier ,agrumes, banane, ananas, pêcher, abricotier, cerisier, noyer, et noisetier.

Selon un mode de réalisation, le procédé susmentionné comprend :
- l'application de la composition phytosanitaire prête à l'emploi décrite ci-dessus sur tout ou partie des végétaux ; ou
- le mélange de la composition phytosanitaire concentrée décrite ci-dessus avec un agent de formulation pour former une composition phytosanitaire finale, suivi de l'application de cette composition phytosanitaire finale sur tout ou partie des végétaux.

Selon un mode de réalisation, l'application de la composition phytosanitaire sur tout ou partie des végétaux consiste en une application sur les feuilles, tiges, fleurs, fruits, tronc et / ou racines ou sur une partie de ceux-ci, de préférence par pulvérisation, l'application sur les racines étant de préférence effectuée par pulvérisation au sol, incorporation mécanique, en mélange avec des engrais, des amendements ou en pré-mix.

La présente invention permet de surmonter les inconvénients de l'état de la technique. Elle fournit plus particulièrement de nouvelles souches de microorganismes antagonistes, plus fiables d'un point de vue industriel, plus sûres d'un point de vue sanitaire et environnemental, et efficaces pour lutter contre les maladies provoquées par les agents pathogènes chez les végétaux.

L'invention repose sur la mise au point par les inventeurs de nouvelles souches de *Saccharomyces cerevisiae* présentant de très bonnes aptitudes phytosanitaires. *Saccharomyces cerevisiae* étant une espèce de levure connue et exploitée depuis des siècles par l'homme pour produire du pain ou de l'alcool, y compris à l'échelle industrielle, son utilisation en tant que microorganisme antagoniste est particulièrement avantageuse.

Ainsi, une lutte efficace contre les agents pathogènes des végétaux et contre les maladies qu'ils provoquent est rendue possible, et ce à partir de produits pas ou peu nocifs pour l'homme et pour l'environnement, et faciles à produire à une échelle industrielle.

Selon des modes de réalisation particuliers, l'invention présente également une, ou de préférence plusieurs, des caractéristiques avantageuses suivantes :
- l'invention permet une longue durée de protection des végétaux (par exemple supérieure à 1 semaine, à 2 semaines ou à 1 mois) et une action polyvalente vis-à-vis d'une pluralité d'agents pathogènes ;
- l'invention est particulièrement applicable dans le domaine de l'agriculture biologique ;
- l'invention permet d'augmenter l'efficacité d'ensemble de la protection phytosanitaire, par la diminution du niveau d'infection et/ou la réduction de rémission d'inoculum ;
- l'invention permet de limiter la quantité des résidus de produits agrochimiques dans ou sur les produits consommables, dans les sols et les eaux lors du traitement de cultures ou d'une plante.

### DESCRIPTION DE MODES DE REALISATION DE L'INVENTION

L'invention est maintenant décrite plus en détail et de façon non limitative dans la description qui suit.

### Types de traitement (curatif ou préventif)

Comme indiqué précédemment, l'invention concerne des procédés et produits pour lutter, à titre préventif ou curatif, contre les agents pathogènes chez les végétaux.

Les végétaux concernés peuvent être tous types de végétaux, et notamment les graminées et dicotylédones, les plantes annuelles, bisannuelles et pérennes, les légumes, les céréales dont le blé, l'orge et le riz, le maïs, le sorgho, le millet, les oléagineux, les protéagineux, les pommes de terre, les betteraves, la canne à sucre, le tabac, les plantes ligneuses, les arbres, fruitiers ou non, les vignes, les végétaux d'ornement...

Selon un mode de mise en oeuvre particulier, les végétaux sont des arbres fruitiers, par exemple des arbres fruitiers à pépins en particulier choisis parmi les pommiers, les poiriers et les agrumes.

Selon un autre mode de mise en oeuvre, les végétaux sont choisis parmi la vigne, les céréales (notamment le blé), le colza, la betterave, la pomme de terre, le haricot, la tomate, le concombre, la laitue ou encore le fraisier.

Le terme de « végétaux » utilisé dans le cadre de la présente invention recouvre à la fois les plantes en tant que telles (par exemple arbres fruitiers...) que des parties isolées de ces plantes, par exemple des fruits ou fleurs récoltés, mais aussi des graines ou semences (matériel de propagation végétale).

Selon un mode de réalisation particulier, les végétaux sont des fruits, par exemple des pommes ou des poires ou des agrumes.

Dans ce qui suit, lorsqu'il est question de mettre en contact les végétaux avec une composition, il faut entendre que cette mise en contact peut s'effectuer sur la totalité ou une partie seulement de la surface des végétaux. Par exemple, lorsque les végétaux sont des plantes complètes, la mise en contact peut s'effectuer sur la totalité des plantes ou sur une ou des parties seulement de celle-ci, telles que par exemple les feuilles, les tiges, les fleurs, les fruits, le tronc et/ou les racines, ou encore une partie seulement de la surface de ces feuilles, tiges, fleurs, fruits, tronc et/ou racines.

Les produits de l'invention permettent de protéger efficacement les végétaux contre des agents pathogènes pendant une période de temps importante, pouvant dépasser un mois, voire plusieurs mois sur fruit conservé à froid. Bien entendu, une application répétée peut être envisagée, à des intervalles à définir par l'utilisateur.

L'invention peut être appliquée à la lutte contre tout type d'agents pathogènes, et notamment des champignons, virus, bactéries, mycoplasmes, spiroplasmes ou viroïdes. La lutte contre les champignons est rendue particulièrement efficace par la présente invention.

A titre d'exemples particuliers d'agents pathogènes, on peut citer notamment les champignons des genres *Alternaria* spp., par exemple *Alternaria solani et Alternaria alternata, Ascochyta* spp., par exemple *Ascochyta fabae* ou *Ascochyta pinodella, Aspergillus spp., notamment Aspergillus niger et Aspergillus fumigatus, Botrytis* spp., par exemple *Botrytis cinerea, Bremia* spp., par exemple *Bremia lactucae, Cercospora* spp., par exemple *Cercospora beticola, Cladosporium* spp., par exemple *Cladosporium allii-cepae, Colletotrichum* spp., par exemple *Colletotrichum graminicola, Cryptosporiopsis spp notamment Cryptosporiopsis malicorticis, Erysiphe* spp., par exemple *Erysiphe graminis ou Erysiphe necator, Fusarium* spp., par exemple *Fusarium oxysporum* et *Fusarium roseum, Gloeosporium* spp., par exemple *Gloeosporium fructigenum, Guignardia* spp., par exemple *Guignardia bidwellii, Helminthosporium* spp., par exemple *Helminthosporium tritici-repentis, Marssonina* spp., par exemple *Marssonina rosae, Monilia* spp., par exemple *Monilia fructigena, Mycosphaerella* spp., par exemple *Mycosphaerella brassicicola, Penicillium* spp., par exemple *Penicillium expansum* ou *Penicillium digitatum, Peronospora* spp., par exemple *Peronospora parasitica, Pezicula* spp. *notamment Pezicula malicorticis, Phragmidium* spp., par exemple *Phragmidium rubi-idaei, Phytophtora* spp., dont *Phytophtora infestans, Plasmopara* spp., dont *Plasmopara viticola, Podosphaera* spp., par exemple *Podosphaera leucotricha, Pseudocercosporella* spp., dont *Pseudocercosporella brassicae, Pseudoperonospora* spp., par exemple *Pseudoperonospora cubensis, Pseudopeziza* spp., par exemple *Pseudopeziza medicaginis, Puccinia* spp., dont *Puccinia graminis, Pythium* spp., *Ramularia* spp., dont *Ramularia betae, Rhizoctonia* spp., par exemple *Rhizoctonia solani, Rhizopus* spp., par exemple *Rhizopus nigricans et Rhizopus stolonifer, Rynchosporium* spp., comme *Rynchosporium secalis, Sclerotinia* spp., comme *Sclerotinia sclerotiorum, Septoria* spp., par exemple *Septoria nodorum* ou *Septoria tritici, Sphaerotheca* spp., comme *Sphaerotheca macularis, Spilocaea spp notamment Spilocaea pomi, Taphrina* spp., par exemple *Taphrina pruni, Trichothecium spp. notamment Trichothecium roseum, Uncinula* spp., par exemple *Uncinula necator, Ustilago* spp., par exemple *Ustilago tritici* et *Venturia* spp., par exemple *Venturia inaequalis.* Des combinaisons des agents pathogènes ci-dessus peuvent aussi être combattues grâce à l'invention.

*Penicillium digitatum, Penicillium expansum, Botrytis cinerea* sont des espèces contre lesquelles l'invention est particulièrement efficace.

Des exemples de bactéries qui affectent les cultures incluent notamment les genres *Corynebacterium, Clavibacter, Curtobacterium, Streptomyces, Pseudomonas, Xanthomonas, Erwinia* spp. et particulièrement *Erwinia amylovora, Erwinia carotovora, Erwinia chrysanthemi.*

Des exemples de virus affectant les cultures sont par exemple les virus de la mosaïque du tabac, ou le virus Y de la pomme de terre.

L'invention propose un procédé de traitement et / ou de protection des végétaux contre les maladies provoquées par les agents pathogènes ci-dessus, par exemple la pourriture grise. Ceci recouvre tout procédé curatif ou préventif, visant à retarder ou empêcher l'apparition de ces maladies, à les éradiquer ou à limiter ou réduire l'ampleur, l'étendue ou les effets de ces maladies.

### Souches de Saccharomyces cerevisiae en tant que microorganismes antagonistes

Par « microorganisme antagoniste », on entend dans le cadre de la présente invention un microorganisme qui est l'antagoniste d'un agent pathogène, en particulier d'un agent pathogène susceptible de provoquer des maladies chez les végétaux (notamment d'un agent pathogène de la liste ci-dessus).

L'invention propose quatre souches de levures *Saccharomyces cerevisiae* qui sont utiles en tant que microorganismes antagonistes, à savoir :
- la souche n°I-3936 déposée le 4 mars 2008 auprès de la CNCM ;
- la souche n°I-3937 déposée le 4 mars 2008 auprès de la CNCM ;
- la souche n°I-3938 déposée le 4 mars 2008 auprès de la CNCM ; et
- la souche n°I-3939 déposée le 4 mars 2008 auprès de la CNCM.

### Utilisation des souches de Saccharomyces cerevisiae de l'invention

L'invention propose l'utilisation des souches de *Saccharomyces cerevisiae* susmentionnées pour protéger ou soigner des végétaux contre des maladies provoquées par des agents pathogènes.

En particulier, pour ce faire, l'invention fournit une composition phytosanitaire comprenant l'une des souches de *Saccharomyces cerevisiae* susmentionnées (ou un mélange de plusieurs de ces souches).

Par « composition phytosanitaire », on entend dans le cadre de la présente invention une composition susceptible de protéger des végétaux contre un ou plusieurs agents pathogènes, et / ou de soigner des végétaux infectés par un ou plusieurs agents pathogènes.

La composition phytosanitaire selon l'invention peut être sous forme concentrée ou sous forme prête à l'emploi. Ladite composition phytosanitaire peut être sous forme sèche, par exemple sous forme de poudre ou granules, ou sous forme liquide, en particulier aqueuse, par exemple sous forme d'une suspension, d'une dispersion, d'un gel, d'une crème, d'une pâte, ou sous forme solide. Dans le cas de la composition prête à l'emploi, la forme liquide aqueuse est préférée, car cette forme est adaptée à la pulvérisation.

En cas de forme sèche, les levures des souches susmentionnées sont de préférence sous forme déshydratée, lyophilisée et / ou encapsulée.

Dans la composition phytosanitaire prête à l'emploi, la matière active (levures *Saccharomyces cerevisiae* des souches susmentionnées) est déjà formulée de manière adaptée à une utilisation sur les plantes. Ainsi la matière active est par exemple mélangée à un véhicule acceptable, tel que par exemple un liquide pour remplir un pulvérisateur de type spray, un engrais, un substrat de culture sous serre... La composition prête à l'emploi peut être sous forme de poudre, mais la forme liquide est préférée.

A la différence de la composition phytosanitaire prête à l'emploi, qui est destinée à être appliquée telle quelle sur les végétaux, la composition phytosanitaire concentrée est destinée à être mélangée avec un agent de formulation (véhicule) par l'utilisateur afin de former une composition finale, prête à l'emploi. De préférence, cet agent de formulation est de l'eau ou une solution aqueuse. Ainsi, typiquement, l'utilisateur dissout des granules (composition phytosanitaire concentrée) dans de l'eau, ou dilue une suspension ou dispersion aqueuse (composition phytosanitaire concentrée) dans de l'eau, afin d'obtenir la composition finale, prête à l'emploi.

Si les levures sont sous forme déshydratée dans la composition phytosanitaire concentrée et si la composition finale est sous forme liquide (typiquement aqueuse), les précautions d'usage pour la réhydratation des levures doivent être respectées ; par exemple la température de l'agent de formulation adapté à la dissolution / réhydratation (typiquement eau ou solution aqueuse) est avantageusement comprise entre 20 et 25°C.

Que la composition phytosanitaire soit concentrée ou prête à l'emploi, elle comprend en général de toute façon un ou plusieurs agents de formulation, solides ou liquides.

Le ou les agents de formulation peuvent être constitués de tout composé ou toute matière inerte permettant de rendre possible, faciliter, ou optimiser le transport, le stockage, la manipulation, l'application et/ou la persistance de la matière active sur les végétaux ou des parties de ceux-ci. De tels agents sont adaptés à l'objectif recherché : conservation des substances actives, tenue en suspension des substances actives lors du stockage ou lors de l'utilisation dans la préparation de la bouillie de traitement, anti-mousse, anti-poussière, adhésion aux végétaux, pénétration dans les tissus, et autres. Ce ou ces agents peuvent être solides, liquides, seuls ou en mélange.

Le ou les agents de formulation peuvent être choisis notamment parmi les agents tensioactifs, dispersants, conservateurs, mouillants, émulsifiants, agents d'adhésion, tampon-pH, nutriments seuls ou en mélange.

La quantité de levures *Saccharomyces cerevisiae* qui est appliquée aux végétaux est définie par l'homme du métier, en fonction notamment de l'agent ou de la pluralité d'agents pathogènes à traiter, du type de végétal, de la souche ou des combinaisons de souches utilisées... La quantité appliquée est de préférence suffisante pour protéger les végétaux contre un agent pathogène, ou pour limiter ou supprimer le développement et les effets de l'agent pathogène présent. Cette quantité peut être déterminée par exemple par des essais en champ (ou, par exemple lorsque les végétaux sont des fruits, par des essais en laboratoire ou en station fruitière sur ces fruits eux-mêmes).

Une composition phytosanitaire prête à l'emploi contient de préférence une quantité de levures des souches susmentionnées comprise entre 10⁴ et 10¹¹ ufc/ml, de préférence entre 10⁵ et 10¹⁰ ufc/ml, de manière plus particulièrement préférée entre 5×10⁵ et 5×10⁹ ufc/ml.

La composition phytosanitaire décrite ci-dessus, sous sa forme finale (prête à l'emploi), peut être appliquée aux végétaux de différentes manières et selon différents protocoles ou programmes de traitements. Dans un mode préféré de mise en oeuvre, la composition est liquide et est appliquée par pulvérisation, notamment par pulvérisation foliaire ou au sol. Dans un mode particulier, la composition est liquide et est appliquée par pulvérisation à des fleurs ou fruits récoltés.

En variante, il est possible d'appliquer la composition sous forme de mélange à des engrais, supports de culture, à l'eau d'arrosage, ou autre. La composition peut ainsi être administrée aux racines par pulvérisation au sol, incorporation mécanique, en mélange avec des engrais, des amendements, en pré-mix, ou autres.

### Autres adjuvants actifs possibles

Dans le cadre du traitement préventif ou curatif de végétaux contre les maladies provoquées par les agents pathogènes, d'autres adjuvants actifs peuvent être utilisés. Par « adjuvant actif » on entend tout composé (différent des levures *Saccharomyces cerevisiae* de souches susmentionnées) susceptible de contribuer à la prévention ou au traitement des maladies des végétaux provoquées par des agents pathogènes, ou susceptible de potentialiser l'effet des levures *Saccharomyces cerevisiae* de souches susmentionnées dans la prévention ou le traitement des maladies des végétaux provoquées par des agents pathogènes.

Selon un mode de réalisation, ces autres adjuvants actifs peuvent être inclus dans la composition phytosanitaire selon l'invention.

Selon un autre mode de réalisation, ces autres adjuvants actifs peuvent être administrés aux végétaux séparément, sous forme d'un traitement complémentaire (de manière séquentielle ou en alternance).

Les autres adjuvants actifs en question comprennent notamment :
1) Tout microorganisme antagoniste différent des levures *Saccharomyces cerevisiae* des souches susmentionnées. On peut utiliser à ce titre tout microorganisme connu en tant qu'antagoniste d'un ou plusieurs agents pathogènes des végétaux.
   De préférence, les microorganismes antagonistes sont choisis parmi les bactéries, les champignons et les levures, de préférence les levures.
   Les microorganismes antagonistes peuvent en particulier être choisis parmi le groupe constitué de *Agrobacterium* spp., *Ampelomyces* spp., *Aureobasidium* spp., *Bacillus* spp., *Bulleromyces* spp., *Candida* spp., *Chaetomium* spp., *Coniothyrium* spp., *Cryptococcus* spp., *Debaryomyces* spp., *Dekkera* spp., *Erwinia* spp., *Exophilia* spp., *Gliocladium* spp., *Hansenula* spp. *Issatchenkia* spp., *Kluyveromyces* spp., *Mariannaea* spp., *Metschnikovia* spp., *Microdochium* spp., *Paecilomyces spp, Penicillium* spp., *Phlebiopsis* spp.; *Pichia* spp., *Pseudomonas* spp., *Pseudozyma* spp., *Pythium spp., Rhodotorula* spp., *Saccharomyces* spp., *Saccharomycopsis spp., Sporobolomyces* spp., *Streptomyces* spp., *Talaromyces* spp., *Trichoderma* spp., *Ulocladium* spp., *Verticilium* spp., *Zygosaccharomyces* spp. et leurs mélanges, et plus particulièrement *Agrobacterium radiobacter, Aureobasidium pullulans, Bacillus subtilis, Bacillus lichenifonnis, Bacillus pumilis, Candida oleophila, Candida saitoana, Candida sake, Candida tenius, Candida utilis, Candida pelliculosa, Coniothyrium minitans, Cryptococcus albidus, Cryptococcus laurentii, Cryptococcus flavescens, Erwinia carotovora, Gliocladium catenalatum, Gliocladium virens, Hanseniaspora uvarum, Kluyveromyces thermotolerance, Metschnikovia fructicola, Metschnikowia pulcherrima, Metschnikowia reukafii, Microdochium dimerum, Paecilomyces fumosoroseus, Penicillium oxalicum, Phlebiopsis gigantean, Pichia anomala, Pichia guilliermondii, Pseudomonas cepacia, Pseudomonas chlororaphis, Pseudomonas fluorescens, Pseudomonas syringae, Pseudozyma flocullosa, Rhodotorula glutinis, Pythium oligandrum, Rhodotorula mucilaginosa, Saccharomyces cerevisiae, Saccharomycopsis schoeni, Streptomyces griseoviridis, Talaromyces flavus, Trichoderma atroviride, Trichoderma harzianum, Trichoderma polysporum, Trichoderma viride, Trichoderma asperellum, Trichoderma gamsi, Ulocladium atrum, Verticilium albo-atrum,* et leurs mélanges.
2) Des sels de calcium, de potassium ou de sodium.
   Des sels particulièrement efficaces en tant qu'adjuvants actifs sont décrits en détail dans le document WO 2006/032530, auquel il est fait référence.
   En particulier les sels chlorure, propionate, sulfate, phosphate, carbonate, bicarbonate, acétate, éthanoate, glycérate, glutamate, érythronate, théonate, ribonate, arabinoate, xylonate, lyxonate, allonate, altronate, gluconate, mannoate, gulonate, idonate, galactonate, talonate, alloheptonate, altroheptonate, glucoheptonate, mannoheptonate, guloheptonate, idoheptonate, galactoheptonate, taloheptonate, tartronate, malate, tartrate, citrate, saccharate, mucate, lactate, lactogluconate, ascorbate, isocitrate et citramalate, de sodium ou de calcium ou de potassium (de préférence de calcium) sont préférés, qu'ils soient sous forme anhydre ou hydratée.
   Le lactate de calcium est particulièrement préféré. Des mélanges des sels ci-dessus sont également possibles.
3) Un agent stimulant choisi parmi le groupe constitué des acides uroniques, des mannanes, du beta-1,3-glucane, de leurs mélanges et dérivés (sels, hydrates...).
   L'acide galacturonique et l'acide glucoronique sont préférés, parmi les acides uroniques.
4) Une substance agrochimique fongicide, antivirale et / ou antibactérienne.
   La substance fongicide peut être choisie par exemple parmi les fongicides agrochimiques organiques ou les fongicides minéraux inorganiques à base de soufre et/ou de cuivre.
   Des exemples de fongicides agrochimiques organiques actuellement disponibles sont notamment les chloronitriles dont le chlorothalonil, les carbamates dont les dithiocarbamates comme le mancozèbe, les phtalimides dont le captane, les sulfamides, les guanidines, les quinones, les quinoléines, les thiadiazines, les anilides, hydroxyanilides, et phénylamides, les imidazolinones, oxazolidinediones, les strobilurines, les cyanoimidazoles, le fluazinam, le dinocap, le sithiofam, les dicarboximides, le fludioxonil, les organophosphorés, le propamocarbe HCl, la diphénylamine, les pyridylamines, les inhibiteurs de la biosynthèse des stérols (IBS), dont les imidazoles, pyrimidines, les hydroxypyrimidines, les anilinopyrimidines, les triazoles, la spiroxamine, les morpholines et les pipéridines, le fenhexamid, l'hymexazol, le zoxamide, le diéthofencarbe, les benzimidazoles, le pencycuron, le quinoxyfene, l'iprovalicarbe, le cymoxanil, le diméthomorphe, les phosphonates, les triazines...

L'invention peut aussi être utilisée en alternance, association ou combinaison avec un ou plusieurs composés éliciteurs de défenses chez les plantes, tels que par exemple l'acide b-aminobutyrique, l'acide 2,6-dichloroisonicotinique, l'acibenzolar-s-methyl ou certains extraits d'algues. Des exemples de tels composés sont notamment la laminarine et les ulvanes.

A cet égard, l'invention propose également un procédé pour prévenir ou freiner le développement de souches de champignons résistantes à une famille d'agents fongicides, caractérisé en ce que les végétaux sont traités avec des levures *Saccharomyces cerevisiae* de souches susmentionnées, comme décrit ci-dessus, en vue de réduire la pression de sélection des souches résistantes à ladite famille d'agents fongicides, ou en ce que le ou les traitements des végétaux avec une substance de ladite famille d'agents fongicides sont alternés ou combinés avec un ou des traitements des végétaux avec des levures *Saccharomyces cerevisiae* de souches susmentionnées, comme décrit ci-dessus.

L'invention propose également un procédé pour prévenir ou freiner le développement de souches de bactéries résistantes à une famille d'agents antibactériens, caractérisé en ce que les végétaux sont traités avec des levures *Saccharomyces cerevisiae* de souches susmentionnées, comme décrit ci-dessus, en vue de réduire la pression de sélection des souches résistantes à ladite famille d'agents antibactériens, ou en ce que le ou les traitements des végétaux avec ledit agent antibactérien sont alternés ou combinés avec un ou des traitements des végétaux avec des levures *Saccharomyces cerevisiae* de souches susmentionnées, comme décrit ci-dessus.

Toutefois, selon un mode de réalisation particulier, la composition phytosanitaire selon l'invention est dépourvue de toute substance agrochimique fongicide, antivirale et / ou antibactérienne toxique pour les levures *Saccharomyces cerevisiae.* Selon un autre mode de réalisation particulier, aucun traitement au moyen d'une quelconque substance agrochimique fongicide, antivirale et / ou antibactérienne n'est appliqué à dose toxique pour les levures *Saccharomyces cerevisiae* avant le traitement par les levures *Saccharomyces cerevisiae* de souches susmentionnées, sauf en respectant le délai d'attente adéquate.

### EXEMPLES

Les exemples suivants illustrent l'invention sans la limiter.

### Exemple 1

On teste l'efficacité de la présente invention contre les maladies fongiques de post-récolte, de la manière suivante.

On pratique sur des pamplemousses trois blessures par fruit. On applique dans chaque blessure une composition phytosanitaire, on laisse sécher, puis on inocule une composition contenant un agent pathogène.

L'agent pathogène utilisé est *Penicillium digitatum,* à une concentration de 5×10⁴ spores par ml.

Au bout de 4 ou 5 jours d'incubation à 20°C, on mesure le diamètre moyen des lésions.

On utilise les 4 types de composition phytosanitaire suivants, avec diverses concentrations de microorganismes (obtenues par dilutions en série dans l'eau distillée stérile à partir d'une culture de microorganismes) :
A : composition contenant la souche n° I-3936 déposée le 4 mars 2008 auprès de la CNCM.
B : composition contenant la souche n°I-3937 déposée le 4 mars 2008 auprès de la CNCM.
C : composition contenant la souche n° I-3938 déposée le 4 mars 2008 auprès de la CNCM.
D : composition contenant la souche n° I-3939 déposée le 4 mars 2008 auprès de la CNCM.

Les souches de levures proviennent de la société Lesaffre International (France).

L'agent pathogène est obtenu par isolement à partir de fruits de pamplemousse pourris ; stockage sur de la gélose inclinée de dextrose - pomme de terre à 4°C ; culture dans des boîtes de gélose inclinée de dextrose - pomme de terre à 25°C ; retrait des spores des bords sporulants d'une culture de 2-3 semaines ; suspension dans de l'eau distillée stérile et ajustement de la concentration en spores avec un hémacytomètre.

Chaque test est moyenné sur 10 fruits.

On mesure l'efficacité des différents traitements préventifs par rapport à un témoin non traité et les résultats sont reportés dans le tableau 1 ci-dessous. Le diamètre des lésions est mesuré à 4 jours, sauf pour les résultats marqués avec un astérisque (*), qui sont obtenus à 5 jours.

**Tableau 1 - prévention de l'infection par Penicillium digitatum sur des pamplemousses**

| Type de traitement | Concentration en levures (en ufc/mL) | Diamètre des lésions (en mm) | Efficacité (%) |
|---|---|---|---|
| Témoin | - | 77,78* | - |
| A | 4,08×10⁷ | 24,07* | 69,0 |
| A | 4,08×10⁸ | 5,56* | 92,9 |
| A | 4,08×10⁹ | 0,00* | 100,0 |
| Témoin | - | 79,63 | - |
| A | 1,10×10⁷ | 24,07 | 69,8 |
| A | 1,10×10⁸ | 18,52 | 76,7 |
| A | 1,10×10⁹ | 5,56 | 93,0 |
| B | 1,10×10⁷ | 20,37 | 74,4 |
| B | 1,10×10⁸ | 25,93 | 67,4 |
| B | 1,10×10⁹ | 12,96 | 83,7 |
| Témoin | - | 83,33 | - |
| C | 3,12×10⁸ | 27,78 | 66,7 |
| C | 3,12×10⁹ | 5,56 | 93,3 |
| C | 3,12×10¹⁰ | 0,00 | 100,0 |
| Témoin | - | 84,44 | - |
| D | 1,10×10⁷ | 1,85 | 97,8 |
| D | 1,10×10⁸ | 1,85 | 97,8 |
| D | 1,10×10⁹ | 0,00 | 100,0 |
| Témoin | - | 90,74 | - |
| D | 1,08×10⁵ | 75,93 | 16,3 |
| D | 1,08×10⁶ | 37,78 | 58,4 |
| D | 1,08×10⁷ | 7,41 | 91,8 |

### Exemple 2

On répète le même type d'expérience que dans l'exemple 1, à la différence près que l'on utilise des pommes au lieu de pamplemousses, et que l'on utilise *Botrytis cinerea* (suspension de spores à la concentration de 10⁶ spores/ml) et *Penicillium expansum* (suspension de spores à la concentration de 10⁵ spores/ml) en tant qu'agents pathogènes. L'évaluation des lésions est effectuée 6 jours après inoculation, les résultats sont moyennés sur 15 fruits à chaque fois.

Les résultats obtenus contre *Botrytis cinerea* sont reportés dans les tableaux 2a et 2b ci-dessous, et les résultats contre *Penicillium expansum* sont reportés dans les tableaux 3a et 3b ci-dessous.

**Tableau 2a - prévention de l'infection par Botrytis cinerea sur des pommes**

| Type de traitement | Concentration en levures (en ufc/mL) | Diamètre des lésions (en mm) | Efficacité (%) |
|---|---|---|---|
| Témoin | - | 21,4 | - |
| A | 10⁷ | 2,3 | 89,18 |
| A | 10⁶ | 9,4 | 55,88 |
| A | 10⁵ | 9,0 | 57,96 |
| B | 10⁷ | 6,9 | 67,64 |
| B | 10⁶ | 13,2 | 38,09 |
| B | 10⁵ | 11,6 | 45,58 |
| C | 10⁷ | 8,4 | 60,87 |
| C | 10⁶ | 11,2 | 47,66 |
| C | 10⁵ | 10,9 | 48,80 |

**Tableau 2b - prévention de l'infection par Botrytis cinerea sur des pommes**

| Type de traitement | Concentration en levures (en ufc/mL) | Diamètre des lésions (en mm) | Efficacité (%) |
|---|---|---|---|
| Témoin | - | 14,1 | - |
| A | 10⁷ | 4,3 | 69,56 |
| A | 10⁸ | 0,0 | 100 |
| D | 10⁵ | 5,6 | 60,57 |
| D | 10⁶ | 3,4 | 76,18 |
| D | 10⁷ | 2,7 | 80,91 |
| D | 5×10⁷ | 0,8 | 94,16 |
| D | 10⁸ | 0,0 | 100 |

**Tableau 3a - prévention de l'infection par Penicillium expansum sur des pommes**

| Type de traitement | Concentration en levures (en ufc/mL) | Diamètre des lésions (en mm) | Efficacité (%) |
|---|---|---|---|
| Témoin | - | 16,9 | - |
| A | 10⁷ | 7,5 | 55,51 |
| A | 10⁶ | 7,3 | 56,69 |
| A | 10⁵ | 12,3 | 27,30 |
| B | 10⁷ | 12,2 | 28,08 |
| B | 10⁶ | 13,0 | 23,36 |
| B | 10⁵ | 14,0 | 17,19 |
| C | 10⁷ | 6,6 | 60,76 |
| C | 10⁶ | 9,4 | 44,62 |
| C | 10⁵ | 11,0 | 34,91 |

**Tableau 3b - prévention de l'infection par Penicillium expansum sur des pommes**

| Type de traitement | Concentration en levures (en ufc/mL) | Diamètre des lésions (en mm) | Efficacité (%) |
|---|---|---|---|
| Témoin | - | 24,0 | - |
| A | 10⁷ | 12,8 | 46,72 |
| A | 10⁸ | 3,8 | 84,0 |
| D | 10⁵ | 15,9 | 33,95 |
| D | 10⁶ | 15,2 | 36,73 |
| D | 10⁷ | 12,0 | 50,05 |
| D | 5.10⁷ | 10,8 | 54,95 |
| D | 10⁸ | 7,2 | 70,12 |

### Exemple 3

On répète le même type d'expérience que dans l'exemple 1, à la différence près que l'on utilise du raisin de table au lieu de pamplemousses, et que l'on utilise *Botrytis cinerea* en tant qu'agent pathogène. En outre, on évalue l'efficacité de la protection par les levures en comptant le nombre de baies de raisin infectées par grappe 7 jours après l'inoculation de l'agent pathogène, de sorte à classer les grappes en 4 catégories : (1) grappe saine ; (2) environ 10 % de la grappe infectée ; (3) environ 25 % de la grappe infectée ; (4) environ 50 % de la grappe infectée ou plus. On attribue la note 0 à la catégorie (1), 0,1 à la catégorie (2), 0,25 à la catégorie (3) et 0,6 à la catégorie (4), de sorte à calculer un dégât moyen.

Les résultats obtenus sur des grappes de raisins non blessées sont reportés dans le tableau 4 ci-dessous, et les résultats obtenus sur des grappes de raisins blessées sont reportés dans le tableau 5 ci-dessous.

**Tableau 4 - prévention de l'infection par Botrytis cinerea sur du raisin de table non blessé**

| Traite^{t} | Concentra° (ufc/mL) | (1) | (2) | (3) | (4) | Dégât moyen | Effic. (%) |
|---|---|---|---|---|---|---|---|
| Témoin | - | 0 | 0 | 2 | 3 | 0,46 | - |
| B | 10⁷ | 1 | 0 | 2 | 2 | 0,34 | 26,1 |
| C | 10⁷ | 0 | 3 | 2 | 0 | 0,16 | 65,2 |
| D | 10⁷ | 1 | 3 | 1 | 0 | 0,11 | 76,1 |
| B | 10⁶ | 0 | 1 | 4 | 0 | 0,22 | 52,2 |
| C | 10⁶ | 0 | 0 | 4 | 1 | 0,32 | 30,4 |
| D | 10⁶ | 0 | 1 | 2 | 2 | 0,36 | 21,7 |

**Tableau 5 - prévention de l'infection par Botrytis cinerea sur du raisin de table blessé**

| Traite^{t} | Concentra° (ufc/mL) | (1) | (2) | (3) | (4) | Dégât moyen | Effic. (%) |
|---|---|---|---|---|---|---|---|
| Témoin | - | 0 | 0 | 4 | 1 | 0,32 | - |
| B | 10⁷ | 0 | 1 | 3 | 1 | 0,29 | 9,4 |
| C | 10⁷ | 0 | 2 | 3 | 0 | 0,19 | 40,6 |
| D | 10⁷ | 0 | 4 | 1 | 0 | 0,13 | 59,4 |
| B | 10⁶ | 0 | 1 | 3 | 1 | 0,29 | 9,4 |
| C | 10⁶ | 0 | 2 | 3 | 0 | 0,19 | 40,6 |
| D | 10⁶ | 0 | 4 | 1 | 0 | 0,13 | 59,4 |

### Exemple 4

Cette expérience a été conduite en conditions réelles, au champ et en station de stockage. Dans un verger de nectarine, variété Fantasia, on a identifié et numéroté 10 arbres dont 5 n'ont pas été traités et 5 l'ont été.

La bouillie de traitement (composition phytosanitaire prête à l'emploi) a été préparée par dispersion dans l'eau à 20°C de granulés de levure séchée à dispersion instantanée, à la concentration de 25 g/L. La souche utilisée pour produire ces granulés était la souche A.

Les fruits ont été traités sur l'arbre, la veille de la récolte, après que les fruits pourris aient été retirés. A la récolte, les plateaux ont été repérés avec le numéro d'arbre, et entrés en cellule réfrigérée.

Les fruits ont été conservés une semaine au froid (4°C), puis conservés à température normale (environ 25°C) pendant 3 jours pour favoriser le développement des maladies. Les notations ont alors été effectuées. Lorsque des taches de *monilia fructigena* ou *botrytis cinerea* étaient présentes, leur nombre et leur diamètre en mm ont été enregistrés, pour chacun des fruits observés.

Au final, ont été calculés : le nombre de fruits sains, le nombre de fruits tachés, le pourcentage de fruits sains et de fruits tachés, le nombre de taches moyen par fruit, le diamètre moyen des taches. L'efficacité selon Abott a été calculée sur les pourcentages de fruits tachés, le nombre moyen de taches, et le diamètre moyen des taches. Les résultats sont présentés dans le tableau 6 ci-dessous.

**Tableau 5 - effet phytosanitaire en conditions de récolte**

| | Fruits récoltés | Fruits tachés | Fruits sains | Taches fongiques | Diamètre des taches en mm |
|---|---|---|---|---|---|
| **Non traités** | | | | | |
| Nombre de fruits | 267 | 202 | 65 | | |
| % | 100 | 75,7 | 24,3 | | |
| Nombre total | | | | 349 | |
| Nombre/fruit taché | | | | 1,73 | |
| Somme des diamètres | | | | | 16309 |
| Diamètre moyen | | | | | 46,7 |

| **Traités** | | | | | |
|---|---|---|---|---|---|
| Nombre | 235 | 114 | 121 | | |
| % | 100 | 48,5 | 51,5 | | |
| Nombre total | | | | 180 | |
| Nombre/fruit taché | | | | 1,58 | |
| Somme des diamètres | | | | | 7779 |
| Diamètre moyen | | | | | 43,2 |
| **Efficacité (%)** | | **35,9** | | **8,6** | **7,5** |

## Revendications

1. Souche de *Saccharomyces cerevisiae,* **caractérisée en ce qu'**elle est choisie parmi la souche n°I-3936 déposée le 4 mars 2008 auprès de la CNCM, la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM.

2. Composition phytosanitaire, comprenant des levures *Saccharomyces cerevisiae* choisies parmi la souche n°I-3936 déposée le 4 mars 2008 auprès de la CNCM, la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM et les mélanges de celles-ci.

3. Composition phytosanitaire selon la revendication 2, comprenant également un ou plusieurs agents de formulation et / ou au moins un adjuvant actif supplémentaire.

4. Composition phytosanitaire selon la revendication 2 ou 3, qui est une composition phytosanitaire concentrée, à formulation sèche solide ou liquide.

5. Composition phytosanitaire selon la revendication 2 ou 3, qui est une composition phytosanitaire prête à l'emploi à formulation sèche ou liquide, de préférence liquide.

6. Composition phytosanitaire selon la revendication 5, comprenant une quantité de levures *Saccharomyces cerevisiae* comprise entre 10⁴ et 10¹¹ ufc/mL, de préférence entre 10⁵ et 10¹⁰ ufc/mL, de manière plus particulièrement préférée entre 5×10⁵ et 5×10⁹ ufc/mL.

7. Procédé de traitement ou de protection de végétaux contre des maladies provoquées par des agents pathogènes, comprenant la mise en contact des végétaux avec des levures *Saccharomyces cerevisiae* choisies parmi la souche n°I-3936 déposée le 4 mars 2008 auprès de la CNCM, la souche n°I-3937 déposée le 4 mars 2008 auprès de la CNCM, la souche n°I-3938 déposée le 4 mars 2008 auprès de la CNCM et la souche n°I-3939 déposée le 4 mars 2008 auprès de la CNCM et les mélanges de celles-ci.

8. Procédé selon la revendication 7, dans lequel les agents pathogènes sont choisis parmi les champignons, virus, bactéries, mycoplasmes, spiroplasmes, viroïdes et combinaisons de ceux-ci, de préférence parmi les champignons.

9. Procédé selon la revendication 7, dans lequel les agents pathogènes sont choisis parmi les microorganismes des genres *Alternaria* spp., notamment *Alternaria solani et Alternaria alternata, Ascochyta spp., notamment Ascochyta fabae ou Ascochyta pinodella, Aspergillus spp., notamment Aspergillus niger et Aspergillus fumigatus, Botrytis spp., notamment Botrytis cinerea, Bremia spp., notamment Bremia* lactucae, *Cercospora* spp., notamment *Cercospora beticola, Cladosporium* spp., notamment *Cladosporium allii-cepae, Colletotrichum spp.,* notamment *Colletotrichum graminicola, Cryptosporiopsis spp notamment Cryptosporiopsis malicorticis, Erysiphe* spp., notamment *Erysiphe graminis ou necator, Fusarium* spp., notamment *Fusarium oxysporum* et *Fusarium roseum, Gloeosporium* spp., notamment *Gloeosporium fructigenum, Guignardia* spp., notamment *Guignardia bidwellii, Helminthosporium* spp., notamment *Helminthosporium tritici-repentis, Marssonina* spp., notamment *Marssonina rosae, Monilia* spp., notamment *Monilia fructigena, Mycosphaerella* spp., notamment *Mycosphaerella brassicicola, Penicillium* spp., notamment *Penicillium expansum* ou *Penicillium digitatum, Peronospora* spp., notamment *Peronospora parasitica, Pezicula spp. notamment Pezicula malicorticis, Phragmidium* spp., notamment *Phragmidium rubi-idaei, Phytophtora* spp., notamment *Phytophtora infestans, Plasmopara spp.,* notamment *Plasmopara viticola, Podosphaera* spp., notamment *Podosphaera leucotricha, Pseudocercosporella* spp., dont *Pseudocercosporella brassicae, Pseudoperonospora* spp., notamment t *Pseudoperonospora cubensis, Pseudopeziza* spp., notamment *Pseudopeziza medicaginis, Puccinia* spp., notamment *Puccinia graminis, Pythium* spp., *Ramularia spp.,* notamment *Ramularia betae, Rhizoctonia* spp., notamment *Rhizoctonia solani, Rhizopus* spp., notamment *Rhizopus nigricans et Rhizopus stolonifer, Rynchosporium* spp., notamment *Rynchosporium secalis, Sclerotinia* spp., notamment *Sclerotinia sclerotiorum, Septoria* spp., notamment *Septoria nodorum* ou *Septoria tritici, Sphaerotheca spp.,* notamment *Sphaerotheca macularis, Spilocaea spp notamment Spilocaea pomi, Taphrina* spp., notamment *Taphrina pruni, Trichothecium spp. notamment roseum, Uncinula* spp., notamment *Uncinula necator, Ustilago* spp., notamment *Ustilago tritici, Venturia* spp., notamment *Venturia inaequalis,* et les combinaisons de ceux-ci, lesdits agents pathogènes étant de préférence choisis parmi *Penicillium digitatum, Penicillium expansum, Botrytis cinerea* et les combinaisons de ceux-ci.

10. Procédé selon l'une des revendications 7 à 9, dans lequel les végétaux sont choisis parmi les graminées, les dicotylédones, les plantes annuelles, bisannuelles et pérennes, les plants de légumes ou les légumes récoltés, les plantes ou arbres à fruits ou les fruits récoltés, les plantes ou arbres à fleurs ou les fleurs récoltées, les céréales, les oléagineux, les protéagineux, les plantes ligneuses, les végétaux d'ornement, et sont notamment choisis parmi les plants ou produits issus de pomme de terre, betterave, canne à sucre, tabac, vigne, blé, colza, orge, riz, maïs, sorgho, millet, soja, haricot, tomate, concombre, laitue, fraisier, pommier, poirier agrumes, banane, ananas, pêcher, abricotier, cerisier, noyer, et noisetier.

11. Procédé selon l'une des revendications 7 à 10, comprenant :
- l'application de la composition phytosanitaire prête à l'emploi selon la revendication 5 ou 6 sur tout ou une partie des végétaux ; ou
- le mélange de la composition phytosanitaire concentrée selon la revendication 4 avec un agent de formulation pour former une composition phytosanitaire finale, suivi de l'application de cette composition phytosanitaire finale sur tout ou une partie des végétaux.

12. Procédé selon la revendication 11, dans lequel l'application de la composition phytosanitaire sur tout ou une partie des végétaux consiste en une application sur les feuilles, tiges, fleurs, fruits, tronc et / ou racines ou sur une partie de ceux-ci, de préférence par pulvérisation, l'application sur les racines étant de préférence effectuée par pulvérisation au sol, incorporation mécanique, en mélange avec des engrais, des amendements ou en pré-mix.

## Patentansprüche

1. *Saccharomyces cerevisiae*-Stamm, **dadurch gekennzeichnet, dass** er ausgewählt ist aus dem am 4. März 2008 bei der CNCM hinterlegten Stamm Nr. I-3936, dem am 4. März 2008 bei der CNCM hinterlegten Stamm Nr. I-3937, dem am 4. März 2008 bei der CNCM hinterlegten Stamm Nr. I-3938 und dem am 4. März 2008 bei der CNCM hinterlegten Stamm Nr. I-3939.

2. Phytosanitäre Zusammensetzung, umfassend *Saccharomyces cerevisiae-*Hefen ausgewählt aus dem am 4. März 2008 bei der CNCM hinterlegten Stamm Nr. I-3936, dem am 4. März 2008 bei der CNCM hinterlegten Stamm Nr. I-3937, dem am 4. März 2008 bei der CNCM hinterlegten Stamm Nr. I-3938 und dem am 4. März 2008 bei der CNCM hinterlegten Stamm Nr. I-3939 und Gemischen davon.

3. Phytosanitäre Zusammensetzung nach Anspruch 2, die des Weiteren ein oder mehrere Formulierungsagenzien und/oder mindestens ein zusätzliches aktives Adjuvans umfasst.

4. Phytosanitäre Zusammensetzung nach Anspruch 2 oder 3, die eine konzentrierte phytosanitäre Zusammensetzung als feste Trockenformulierung oder als Flüssigformulierung ist.

5. Phytosanitäre Zusammensetzung nach Anspruch 2 oder 3, die eine gebrauchsfertige phytosanitäre Zusammensetzung als Trockenformulierung oder als Flüssigformulierung, bevorzugt als Flüssigformulierung ist.

6. Phytosanitäre Zusammensetzung nach Anspruch 5, umfassend eine Menge an *Saccharomyces cerevisiae*-Hefen*,* die im Bereich zwischen 10⁴ und 10¹¹ cfu/ml [Colony Forming Units/Milliliter], bevorzugt zwischen 10⁵ und 10¹⁰ cfu/ml, besonders bevorzugt zwischen 5x10⁵ und 5×10⁹ cfu/ml liegt.

7. Verfahren zur Behandlung oder zum Schutz von Pflanzen gegen durch pathogene Agenzien hervorgerufene Krankheiten, umfassend das Inkontaktbringen der Pflanzen mit *Saccharomyces cerevisiae*-Hefen*,* die ausgewählt sind aus dem am 4. März 2008 bei der CNCM hinterlegten Stamm Nr. I-3936, dem am 4. März 2008 bei der CNCM hinterlegten Stamm Nr. I-3937, dem am 4. März 2008 bei der CNCM hinterlegten Stamm Nr. I-3938 und dem am 4. März 2008 bei der CNCM hinterlegten Stamm Nr. I-3939 und Gemischen dieser Stämme.

8. Verfahren nach Anspruch 7, wobei die pathogenen Agenzien ausgewählt sind aus Pilzen, Viren, Bakterien, Mycoplasmen, Spiroplasmen, Viroiden und Kombinationen davon, bevorzugt aus Pilzen.

9. Verfahren nach Anspruch 7, wobei die pathogenen Agenzien ausgewählt sind aus Mikroorganismen der Gattungen *Alternaria spp.,* insbesondere *Alternaria solani* und *Alternaria alternata, Ascochyta* spp., insbesondere *Ascochyta fabae* oder *Ascochyta pinodella, Aspergillus* spp., insbesondere *Aspergillus niger* und *Aspergillus fumigatus, Botrytis* spp., insbesondere *Botrytis cinerae, Bremia* spp., insbesondere *Bremia lactucae, Cercospora* spp., insbesondere *Cercospora beticola, Cladosporium* spp., insbesondere *Cladosporium alliicepae, Colletotrichum* spp., insbesondere *Colletotrichum graminicola, Cryptosporiopsis* spp., insbesondere *Cryptosporiopsis malicorticis, Erysiphe* spp., insbesondere *Erysiphe graminis* oder *necator, Fusarium* spp., insbesondere *Fusarium oxysporum* und *Fusarium roseum, Gloeosporium* spp., insbesondere *Gloeosporium fructigenum, Guignardia* spp., insbesondere *Guignardia bidwellii, Helminthosporium* spp., insbesondere *Helminthosporium tritici-repentis, Marssonina* spp., insbesondere *Marssonina rosae, Monilia* spp., insbesondere *Monilia fructigena, Mycosphaerella* spp., insbesondere *Mycosphaerella brassicicola, Penicillium* spp., insbesondere *Penicillium expansum* oder *Penicillium digitatum, Peronospora* spp., insbesondere *Peronospora parasitica, Pezicula* spp. insbesondere *Pezicula malicorticis, Phragmidium* spp., insbesondere *Phragmidium rubi-idaei, Phytophtora* spp., insbesondere *Phytophtora infestans, Plasmopara* spp., insbesondere *Plasmopara viticola, Podosphaera* spp., insbesondere *Podosphaera leucotricha, Pseudocercosporella* spp., von diesen *Pseudocercosporella brassicae, Pseudoperonospora* spp., insbesondere *Pseudoperonospora cubensis, Pseudopeziza* spp., insbesondere *Pseudopeziza medicaginis, Puccinia* spp., insbesondere *Puccinia graminis, Pythium* spp., *Ramularia* spp., insbesondere *Ramularia betae, Rhizoctonia* spp., insbesondere *Rhizoctonia solani, Rhizopus* spp., insbesondere *Rhizopus nigricans* und *Rhizopus stolonifer, Rynchosporium* spp., insbesondere *Rynchosporium secalis, Sclerotinia* spp., insbesondere *Sclerotinia sclerotiorum, Septoria* spp., insbesondere *Septoria nodorum* oder *Septoria tritici, Sphaerotheca* spp., insbesondere *Sphaerotheca macularis, Spilocaea* spp., insbesondere *Spilocaea pomi, Taphrina* spp., insbesondere *Taphrina pruni, Trichothecium* spp. insbesondere *roseum, Uncinula* spp., insbesondere *Uncinula necator, Ustilago* spp., insbesondere *Ustilago tritici, Venturia* spp., insbesondere *Venturia inaequalis,* und Kombinationen aus diesen, wobei die pathogenen Agenzien vorzugsweise ausgewählt sind aus *Penicillium digitatum, Penicillium expansum, Botrytis cinerea* und Kombinationen aus diesen.

10. Verfahren nach einem der Ansprüche 7 bis 9, wobei die Pflanzen ausgewählt sind aus Gräsern, zweikeimblättrigen Pflanzen, einjährigen Pflanzen, zweijährigen Pflanzen und ausdauernden Pflanzen, Gemüsepflanzen oder geerntetem Gemüse, Obstpflanzen oder Obstbäumen oder geerntetem Obst, Blütenpflanzen oder Blütenbäumen oder geernteten Blüten, Getreide, Ölpflanzen, Eiweißpflanzen, Holzgewächsen, Zierpflanzen, und vorzugsweise ausgewählt sind aus Pflanzen oder Produkten, die aus Kartoffel, Rübe, Zuckerrohr, Tabak, Weinrebe, Weizen, Raps, Gerste, Reis, Mais, Sorghum, Hirse, Soja, Bohne, Tomate, Gurke, Kopfsalat, Erdbeerpflanze, Apfelbaum, Birnbaum, Agrumen, Banane, Ananas, Pfirsichbaum, Aprikosenbaum, Kirschbaum, Walnussbaum und Haselnussbaum hervorgegangen sind.

11. Verfahren nach einem der Ansprüche 7 bis 10, umfassend:
- Aufbringen der gebrauchsfertigen phytosanitären Zusammensetzung gemäß Anspruch 5 oder 6 auf die Pflanze als Ganzes oder auf einen Teil der Pflanze; oder
- Mischen der konzentrierten phytosanitären Zusammensetzung gemäß Anspruch 4 mit einem Formulierungsagens, um eine finale phytosanitäre Zusammensetzung zu bilden, gefolgt von dem Aufbringen der finalen phytosanitären Zusammensetzung auf die Pflanze als Ganzes oder auf einen Teil der Pflanze.

12. Verfahren nach Anspruch 11, wobei das Aufbringen der phytosanitären Zusammensetzung auf die Pflanze als Ganzes oder einen Teil der Pflanze in einem Aufbringen auf Blätter, Stängel, Blüten, Früchte, Stamm und/oder Wurzeln oder auf einen Teil von diesen besteht, vorzugsweise mittels Zerstäubung, wobei das Aufbringen auf die Wurzeln vorzugsweise mittels Zerstäuben auf dem Boden, mechanischer Einarbeitung, in Mischung mit Düngern, Bodenverbesserungsmitteln oder in Form einer Fertigmischung erfolgt.

## Claims

1. *Saccharomyces cerevisiae* strain, **characterized in that** it is selected from strain No. 1-3936 deposited on March 4^{th} 2008 at the CNCM, strain No. 1-3937 deposited on March 4^{th} 2008 at the CNCM, strain No. I-3938 deposited on March 4^{th} 2008 at the CNCM, and strain No. I-3939 deposited on March 4^{th} 2008 at the CNCM.

2. A phytosanitary composition comprising *Saccharomyces cerevisiae* yeasts selected from strain No. I-3936 deposited on March 4^{th} 2008 at the CNCM, strain No. 1-3937 deposited on March 4^{th} 2008 at the CNCM, strain No. I-3938 deposited on March 4^{th} 2008 at the CNCM, and strain No. I-3939 deposited on March 4^{th} 2008 at the CNCM and mixtures thereof.

3. The phytosanitary composition according to claim 2, further comprising one or more formulation agents and/or at least one additional active adjuvant.

4. The phytosanitary composition according to claim 2 or 3, which is a concentrated phytosanitary composition with a dry, solid or liquid formulation.

5. The phytosanitary compositon according to claim 2 or 3, which is a ready-to-use phytosanitary composition with a dry or liquid formulation, preferably liquid formulation.

6. The phytosanitary composition according to claim 5, comprising an amount of *Saccharomyces cerevisiae* yeasts comprised between 10⁴ and 10¹¹ cfu/mL, preferably between 10⁵ and 10¹⁰ cfu/mL, more preferably between 5x10⁵ and 5x10⁹ cfu/mL.

7. A method for treating or protecting plants against diseases caused by pathogens, comprising contacting plants with *Saccharomyces cerevisiae* yeasts selected from strain No. 1-3936 deposited on March 4^{th} 2008 at the CNCM, strain No. 1-3937 deposited on March 4^{th} 2008 at the CNCM, strain No. I-3938 deposited on March 4^{th} 2008 at the CNCM, and strain No. 1-3939 deposited on March 4^{th} 2008 at the CNCM, and mixtures thereof.

8. The method according to claim 7, wherein the pathogens are selected from fungi, viruses, bacteria, mycoplasms, spiroplasms, viroids and combinations thereof, preferably fungi.

9. The method according to claim 7, wherein the pathogens are selected from microorganisms of the genera *Alternaria spp.,* in particular *Alternaria solani* and *Alternaria alternata, Ascochyta spp.,* in particular *Ascochyta fabae* or *Ascochyta pinodella, Aspergillus spp.,* in particular *Aspergillus niger* and *Aspergillus fumigatus, Botrytis spp.,* in particular *Botrytis cinerea, Bremia spp.,* in particular *Bremia lactucae, Cercospora spp.,* in particular *Cercospora beticola, Cladosporium spp.,* in particular *Cladosporium allii-cepae, Colletotrichum spp.,* in particular *Colletotrichum graminicola, Cryptosporiopsis spp.,* in particular *Cryptosporiopsis malicorticis, Erysiphe spp.,* in particular *Erysiphe graminis* or *necator, Fusarium spp.,* in particular *Fusarium oxysporum* and *Fusarium roseum, Gloeosporium spp.,* in particular *Gloeosporium fructigenum, Guignardia spp.,* in particular *Guignardia bidwellii, Helminthosporium spp.,* in particular *Helminthosporium tritici-repentis, Marssonina spp.,* in particular *Marssonina rosae, Monilia spp.,* in particular *Monilia fructigena, Mycosphaerella spp.,* in particular *Mycosphaerella brassicicola, Penicillium spp.,* in particular *Penicillium expansum* or *Penicillium digitatum, Peronospora spp.,* in particular *Peronospora parasitica, Pezicula spp.,* in particular *Pezicula malicorticis, Phragmidium spp.,* in particular *Phragmidium rubi-idaei, Phytophtora spp.,* in particular *Phytophtora infestans, Plasmopara spp.,* in particular *Plasmopara viticola, Podosphaera spp.,* in particular *Podosphaera leucotricha, Pseudocercosporella spp.,* including *Pseudocercosporella brassicae, Pseudoperonospora spp.,* in particular *Pseudoperonospora cubensis, Pseudopeziza spp.,* in particular *Pseudopeziza medicaginis, Puccinia spp.,* in particular *Puccinia graminis, Pythium spp., Ramularia spp.,* in particular *Ramularia betae, Rhizoctonia spp.,* in particular *Rhizoctonia solani, Rhizopus spp.,* in particular *Rhizopus nigricans* and *Rhizopus stolonifer, Rynchosporium spp.,* in particular *Rynchosporium secalis, Sclerotinia spp.,* in particular *Sclerotinia sclerotiorum, Septoria spp.,* in particular *Septoria nodorum* or *Septoria tritici, Sphaerotheca spp.,* in particular *Sphaerotheca macularis, Spilocaea spp.,* in particular *Spilocaea pomi, Taphrina spp.,* in particular *Taphrina pruni, Trichothecium spp.,* in particular *roseum, Uncinula spp.,* in particular *Uncinula necator, Ustilago spp.,* in particular *Ustilago tritici, Venturia spp.,* in particular *Venturia inaequalis,* and combinations thereof, said pathogens being preferably selected from *Penicillium digitatum, Penicillium expansum, Botrytis cinerea* and combinations thereof.

10. The method according to one of claims 7 to 9, wherein the plants are selected from grasses, dicotyledonous plants, annual, biannual and perennial plants, vegetable seedlings or harvested vegetables, fruit plants or trees or harvested fruit, flower plants or trees or harvested flowers, cereals, oleaginous plants, proteaginous plants, ligneous plants, ornamental plants and are notably selected from seedlings or products from potatoes, beetroots, sugar cane, tobacco, vine, wheat, rapeseed, barley, rice, maize, sorghum, millet, soya bean, beans, tomatoes, cucumbers, lettuces, strawberry plants, apple trees, pear trees, citrus fruit, banana, pineapple, peach trees, apricot trees, cherry trees, walnut trees and hazelnut trees.

11. The method according to one of claims 7 to 10, comprising:
- applying the read-to-use phytosanitary composition according to claim 5 or claim 6 on all or a part of the plants; or
- mixing the concentrated phytosanitary composition according to claim 4 with a formulation agent in order to form a final phytosanitary composition, followed by applying this final phytosanitary composition on all or a part of the plants.

12. The method according to claim 11, wherein the application of the phytosanitary composition on all or a part of the plants consists in an application on the leaves, stems, flowers, fruit, trunk and/or roots or on a part thereof, preferably by spraying, the application on the roots being preferably carried out by ground spraying, mechanical incorporation, by mixing with fertilizers, enriching agents or as a premix.
